Europäisches Patentamt

European Patent Office

Office européen des brevets

(19)

(11) **EP 0 861 291 B1**

(12) **FASCICULE DE BREVET EUROPEEN**

(45) Date de publication et mention
de la délivrance du brevet:
**10.05.2000 Bulletin 2000/19**

(51) Int Cl.⁷: **C08L 5/00**, C08L 5/14

(21) Numéro de dépôt: **96939123.4**

(22) Date de dépôt: **14.11.1996**

(86) Numéro de dépôt international:
**PCT/FR96/01803**

(87) Numéro de publication internationale:
**WO 97/18263 (22.05.1997 Gazette 1997/22)**

(54) **ADJUVANT DE TEXTURE COMPORTANT UNE GOMME XANTHANE ET UN GALACTOMANNANE, COMPOSITION L'INCORPORANT, ET UTILISATION POUR EPAISSIR UNE PHASE AQUEUSE**

GERÜSTSTOFF FÜR TEXTUR ENTHALTEND EINEN XANTHANGUMMI UND EINEN GALACTOMANNAN, DIESEN ENTHALTENDE ZUSAMMENSETZUNG UND VERWENDUNG ZUM EINDICKEN EINER WÄSSRIGE PHASE

TEXTURE ADDITIVE COMPRISING A XANTHAN GUM AND A GALACTOMANNAN, COMPOSITION CONTAINING SAME, AND USE THEREOF FOR THICKENING AN AQUEOUS PHASE

(84) Etats contractants désignés:
**AT BE CH DE DK ES FI FR GB GR IE IT LI LU MC NL PT SE**

(30) Priorité: **15.11.1995  FR 9513511**
**15.11.1995  FR 9513510**

(43) Date de publication de la demande:
**02.09.1998  Bulletin 1998/36**

(73) Titulaire: **RHODIA CHIMIE**
**92408 Courbevoie Cédex (FR)**

(72) Inventeurs:
• **DOUBLIER, Jean-Louis**
**F-44800 Saint-Herblain (FR)**
• **SCHORSCH, Catherine**
**F-92700 Colombes (FR)**
• **KNIPPER, Magali**
**F-75018 Paris (FR)**

(74) Mandataire: **Ricalens, François**
**RHODIA CHIMIE**
**Direction de la Propriété Industrielle**
**25, quai Paul Doumer**
**92408 Courbevoie Cédex (FR)**

(56) Documents cités:
**EP-A- 0 001 192          EP-A- 0 139 913**
**EP-A- 0 602 990          US-A- 3 996 389**

• **PATENT ABSTRACTS OF JAPAN vol. 018, no. 242 (C-1197), 10 Mai 1994 & JP 06 030714 A (MEIJI SEIKA KAISHA LTD), 8 Février 1994,**
• **CARBOHYDRATE POLYMERS, vol. 16, no. 3, 1991, ESSEX GB, pages 239-252, XP000215808 TAKO M.: "Synergistic interaction between Xanthan and Tara-Bean gum"**
• **CARBOHYDRATE RESEARCH, vol. 138, 1985, GB, pages 207-213, XP002007569 TAKO M. ET AL.: "Synergistic interaction between xanthan and guar gum"**
• **MACROMOLECULES, vol. 27, no. 15, 1994, US, pages 4204-4211, XP000456656 ANNABLE P. ET AL.: "Interaction in Xanthan-Glucomannan mixtures and the influence of electrolyte"**

Remarques:
Le dossier contient des informations techniques présentées postérieurement au dépôt de la demande et ne figurant pas dans le présent fascicule.

## Description

La présente invention est issue d'une recherche menée en collaboration entre l'Institut National de Recherche Agronomique (INRA) et Rhône-Poulenc. Elle a pour objet un adjuvant de texture comportant une gomme xanthane et un galactomannane, une composition l'incorporant, et l'utilisation de la composition pour épaissir une phase aqueuse. Elle concerne plus particulièrement l'association de gommes xanthane avec des galactomannanes spécifiques.

Elle a également pour objet un adjuvant de gélification, un procédé de gélification et des compositions en découlant.

Avant de développer les caractéristiques de la présente invention il convient de rappeler quelques données sur les associations de galactomannane et de polyoside souvent désignées par gomme xanthane.

Diverses industries, telles que les industries cosmétique, tinctoriale, alimentaire, celle du pétrole et celle des produits d'entretien recherchent des agents de texture qui modifient les propriétés rhéologiques des phases liquides et notamment des phases aqueuses. Les meilleures propriétés connues à ce jour sont obtenues à partir de produits coûteux ou dont la production est soumise à des aléas climatiques.

Un des buts poursuivis par l'industrie alimentaire est de remplacer les agents de texture usuels qui souvent apportent des calories (amidon, gélatine, ...), par des agents non métabolisables et n'apportant de ce fait pas d'énergie.

Les modifications de rhéologie des phases aqueuses sont souvent obtenues par des dérivés de glucides d'origine végétale ou animale.

Ainsi, les galactomannanes sont des polyosides non ioniques extraits de l'albumen de graines de légumineuses dont ils constituent le glucide de réserve.

La gomme de caroube est extraite des graines de caroubier *(Ceratonia siliqua),* qui est un arbre à feuillage persistant originaire de Syrie et d'Asie mineure, mais cultivé sur tout le littoral méditerranéen.

La gomme guar, qui n'est apparue qu'après la seconde guerre mondiale pour pallier une pénurie de caroube, provient des graines de guar *(Cyamopsis tetragonolobus),* plante annuelle initialement cultivée en Inde et au Pakistan et également produite aux Etats-Unis depuis 1950.

Par rapport au caroubier, le guar présente trois avantages agronomiques :

- un temps de maturité plus réduit (un an au lieu de cinq) ;
- une taille de graines plus petite, qui permet une récolte mécanisée ;
- un rendement des graines plus élevé (50 % environ).

Le procédé de fabrication de ces gommes consiste à broyer les albumens des graines décortiquées pour obtenir la farine (95 % de galactomannane). La farine peut être commercialisée telle quelle ou après traitement(s).

Les galactomannanes sont des macromolécules constituées d'une chaîne principale d'unités D-mannopyranose liées en $\beta(1\text{-}4)$, portant des branchements latéraux constitués d'une seule unité D-galactopyranose liée en $\alpha(1\text{-}6)$ à la chaîne principale. Les différents galactomannanes se distinguent par la proportion d'unités $\alpha$-D-galactopyranose présentes dans le polymère.

Le rapport mannose/galactose (M/G) est de l'ordre de 2 pour la gomme guar, et de 4 pour la gomme de caroube, ce qui signifie que la molécule de guar possède en moyenne deux fois plus de branchements. Cependant, au sein d'un même échantillon, ce rapport peut varier selon les fractions. La connaissance du rapport M/G constitue un des moyens de caractériser l'échantillon bien qu'il n'informe pas sur la distribution statistique des résidus galactosyles sur la chaîne principale.

La masse moléculaire moyenne en poids $\overline{M}_p$ est déterminée directement par la diffusion de la lumière ou à partir de la viscosité intrinsèque en utilisant un étalonnage selon Robinson et alii (Robinson, G., Ross-Murphy, S.B., Morris, E.R., 1982, "Viscosity-Molecular weight relationships, intrinsic chain flexibility and dynamic solution properties of guar Galactomannan" Carbohydrate research, **107** 17-32) ; elle est de l'ordre de $1\text{-}2\ 10^6$ mole par gramme pour le guar comme pour la caroube.

Les galactomannanes ci-dessus (guar et caroube), qui dans l'eau forment des solutions macromoléculaires, ont à titre indicatif des viscosités intrinsèques :

. de l'ordre de 1 à 1,5 $m^3$/Kg pour la caroube ;
. et pouvant varier de 0,2 à 2 $m^3$/Kg dans le cas du guar.

En solution dans l'eau le régime dilué correspond à une concentration telle que toutes les macromolécules sont indépendantes les unes des autres. La concentration à partir de laquelle les chaînes commencent à s'enchevêtrer est appelée concentration limite de recouvrement et notée C*. Pour les galactomannanes selon la présente invention elle se situe à une valeur telle que le produit C*[η] soit égal à 1. Le taux d'enchevêtrements des chaînes augmente alors avec la concentration en polymères. On définit une concentration C** à la limite du régime dit semi-dilué (la zone entre les valeurs de concentration C* et C** correspondant à un régime intermédiaire).

Les solutions semi-diluées de galactomannane (C ≥ C** et avantageusement C ≥ C**) présentent un comportement non-thixotrope (indépendant du temps) et rhéofluidifiant (la viscosité apparente diminue quand la vitesse de cisaillement augmente). Un comportement newtonien n'est observé qu'aux faibles vitesses de cisaillement.

Quant à elle, la gomme xanthane est un polyoside exocellulaire anionique synthétisé notamment par une bactérie *Xanthomonas campestris.*

La gomme xanthane est le polyoside d'origine microbienne de loin le plus utilisé dans le secteur alimentaire (préparations lactées, crèmes glacées, assaisonnements, sauces, boissons ...). Ses applications industrielles sont nombreuses dans des domaines comme les peintures, les teintures pour textiles, les cosmétiques, les produits d'entretien ou encore la récupération assistée des pétroles.

Le motif de base du polymère est composé de cinq résidus osidiques : la chaîne principale est constituée d'unités de D-glucose, reliées entre elles par des liaisons β(1-4). Il s'agit d'une structure identique à celle de la cellulose. Un glucose sur deux est substitué en C3 par une chaîne triosidique anionique, comportant un acide glucuronique et deux mannoses. Le premier mannose est partiellement acétylé et le mannose terminal peut porter un acide pyruvique sous forme d'acétal avec les carbones 4 et 6. Ces taux d'acétate et de pyruvates sont variables suivant les souches utilisées et les conditions du milieu de culture. Pour le xanthane usuel, le degré de substitution en pyruvate est de 0,3-0,5 alors que celui en acétate est souvent proche de 1.

La molécule de xanthane adopte une conformation désordonnée ou ordonnée, selon les paramètres du milieu et notamment la température et la force ionique, dans des conditions qui seront précisées ultérieurement. Le passage entre la conformation ordonnée hélicoïdale et une conformation désordonnée plus linéaire (dite "en pelote") se traduirait aussi par une simple augmentation de l'ordre de 10 % du rapport axial (longueur/diamètre) de la molécule. La température de cette transition dépend de la force ionique.

On a proposé depuis quelques lustres d'utiliser des mélanges de xanthane et de gomme caroube pour obtenir des produits épaississant et/ou gélifiant.

Toutefois compte tenu des aléas climatiques et de la grande volatilité des cours de la caroube les débouchés d'une telle association sont peu évidents.

Les associations de la gomme xanthane avec d'autres galactomannanes moins chers et plus réguliers tel que le guar, sont réputées trop faibles pour avoir un effet aussi marqué.

C'est pourquoi un des buts de la présente invention est de fournir des adjuvants de texture qui puissent remplacer les associations de la xanthane avec les dérivés de galactomannane issus de la caroube.

Un autre but poursuivi est de remplacer les agents de texture usuels qui souvent apportent des calories (amidon, gélatine, ...) par des agents non métabolisables et n'apportant de ce fait pas d'énergie.

Un autre but poursuivi est de fournir des gels ou des pseudogels dont le point de fusion soit au voisinage de la température humaine, c'est-à-dire compris dans l'intervalle 30° à 50°C.

Le document **Carbohydrate Research, 138 (1985) 207 - 213** décrit une série de mélanges aqueux de gomme xanthane (natif ou déscétylé) et de gomme guar, pouvant former des gels à des températures basses (0°C).

La présence d'un sel tel que NaCl permet de réduire la viscoélasticité du mélange de gommes.

Les mélanges de gommes dans D2 ne permettent en aucun cas d'obtenir des gels dont le point de fusion est compris dans l'intervalle 30°C à 50°C.

Ces buts et d'autres qui apparaîtront par la suite sont atteints au moyen d'un adjuvant de texture comportant :

une gomme xanthane et
un galactomannane
et dans lequel au moins une des conditions suivantes est remplie :
ledit galactomannane de rapport mannose/galactose au plus égal à 10/3, présente une viscosité intrinsèque [η] ([èta]) au moins égale à environ 1,5 m³/Kg;

il comporte en outre : un sel dissocié.

Avec la première des conditions on privilégie le caractère épaississant alors qu'avec la seconde on privilégie la formation de gel. Les compositions selon l'invention présentent en outre des propriétés de thixotropie intéressantes.

L'association des deux conditions conduit à des gels particulièrement fermes et de point de fusion assez élevé que l'on peut moduler en jouant sur les rapports entre les divers composants.

Pour obtenir des adjuvants conduisant à de fortes propriétés texturantes, il est souhaitable que ledit galactomannane présente une viscosité intrinsèque [η] soit au moins égale à 1,5 m³/Kg.

Dans le domaine considéré (rapport mannose/galactose au plus égal à 10/3), pour obtenir des augmentations élevées de fermeté, il est souhaitable que ledit galactomannane présente un rapport massique (ou molaire, puisqu'il n'y a pas de différence significative entre les masses moléculaire des deux motifs) mannose/galactose au moins égal à environ 3/2 ; avantageusement au moins égal à 5/3 ; de préférence à 1,8 , plus préférentiellement à 2.

Selon une mise en oeuvre particulièrement avantageuse de la présente invention, le rapport massique entre ledit xanthane et ledit galactomannane (X/G) est choisi de manière à être au moins égal à 1/500, avantageusement à 1/100, de préférence à 1/50 ; il est également souhaitable que le rapport massique entre ledit xanthane et ledit galactomannane (X/G) soit au plus égal à 2, avantageusement à 1, de préférence (notamment pour des raisons économiques)à 1/3.

Les qualités de texture des compositions selon la présente invention croissent avec le taux d'acide pyruvique. Ainsi, il est souhaitable que ladite gomme xanthane présente un taux élevé d'acide pyruvique. Par haut taux d'acide pyruvique on entend un taux au moins égal à 1,5, avantageusement à 2,5 %, de préférence compris entre 2 et 6 %. Il convient de noter qu'il est difficile d'avoir un taux supérieur à environ 7 %.

Il en va de même avec la désacétylation ; aussi, il est préférable que ladite gomme xanthane soit, au moins partiellement, desacétylée.

L'adjuvant de texture selon la présente invention est potentialisé par la présence d'un sel dissocié. Cette amélioration des propriétés texturantes ne pouvait guère être déduite des connaissances de l'homme du métier.

En effet, parmi les interactions de tels mélanges au moins ternaires [galactomannane avec xanthane et sel (s) dissocié(s)], les seules connues et avérées à ce jour, le sont avec la gomme de caroube (rapport mannose/galactose (M/G) de l'ordre de 4).

Les interactions dans les mélanges selon la présente invention paraissent très différentes de celles décrites antérieurement. C'est ce que semble montrer le fait que les effets du sel dissocié sur les mélanges décrits dans l'état antérieur de la technique pour la caroube soient notablement plus faibles, voire même inverses, que ceux qui sont constatés sur les mélanges selon la présente invention.

Ainsi, selon une mise en oeuvre particulièrement avantageuse de la présente invention, ledit sel est un sel ou un mélange de sels bien dissociés. Quoique les divalents, et notamment les alcalinoterreux [avantageusement de rang atomique élevé (c'est-à-dire dont le rang est au moins égal à celui du sodium)], puissent être utilisés, il est souhaitable de faire appel à des sels à cation(s) monovalent(s). En outre, il est également souhaitable que ledit sel soit un sel ou un mélange de sels à anion(s) monovalent(s). Cation(s) et anion(s) peuvent être organiques ou minéraux à condition que leur(s) sel(s) soi(en)t bien dissocié(s). Comme anion organique, on peut citer les carboxylates, y compris les diacides tels l'acide glutamique, et les sulfonates.

Avantageusement ledit sel est un sel ou un mélange de sels dont l'anion est choisi parmi les halogénures ou leur mélanges, avantageusement de rang atomique élevé (c'est-à-dire dont le rang est au moins égal au chlorure).

De préférence, ledit sel est un sel ou un mélange de sel dont le cation est choisi parmi les alcalins et leur mélanges, avantageusement de rang atomique élevé (c'est-à-dire dont le rang est au moins égal à celui du sodium).

Comme autre possibilité avantageuse, le cation peut être choisi parmi les sels d'ammonium quaternaire.

Les conditions de cette mise en mélange (temps, pH, etc.) doivent être suffisamment douces pour que l'on évite la dégradation des constituants et notamment des gommes guar et xanthane. Ainsi il est préférable que la durée de mélange ne dépasse pas un quart de journée, avantageusement 2 heures, de préférence 1/2 heure aux températures maximales spécifiées ci-dessus. Les durées peuvent être supérieures si l'on se place dans la partie basse des fourchettes ci-dessus. A titre indicatif la durée minimale de mise en mélange aux températures ci-dessus varie en général entre 1/24 et 1/4 d'heure.

Pour obtenir les meilleurs résultats de fermeté, c'est-à-dire dont le module G' (module de stockage ou module élastique mesuré à 1 rad par seconde, à 25°C et à une concentration totale en xanthane et en galactomannane de 0,5 %) présente une valeur au moins égale à 3 Pa, avantageusement à 5, de préférence à 7 Pa, il est plus aisé de procéder comme cela est décrit plus loin.

Selon un mode préféré de la présente invention, en présence de sel dissocié, le module élastique G' du mélange est au moins égal à deux fois de préférence à cinq fois la valeur obtenue en mélangeant à 25°C une solution de galactomannane avec une solution de xanthane pour obtenir une composition semblable.

Selon une mise en oeuvre particulièrement avantageuse de la présente invention, le rapport massique entre ledit xanthane et ledit galactomannane (X/G) est choisi entre 1/500 et 2, avantageusement entre 1/100 et 1, de préférence entre 1/50 et 1/3.

Les qualités de texture des compositions selon la présente invention croissent avec le taux d'acide pyruvique. Ainsi, il est souhaitable que ladite gomme xanthane présente un taux élevé d'acide pyruvique. Par haut taux d'acide pyruvique on entend un taux au moins égal à 1,5, avantageusement à 2,5 %, de préférence compris entre 2 et 6 %. Il convient de noter qu'il est difficile d'avoir un taux supérieur à environ 7 %.

Il en va de même avec la désacétylation ; aussi, il est préférable que ladite gomme xanthane soit, au moins partiellement, désacétylée.

Pour obtenir les propriétés visées par la présente invention en présence, fût-elle très faible, de sel dissocié, il est souhaitable que l'addition des composants soit réalisée à une température au moins égale à 50°C, avantageusement à 60°C, de préférence à 80°C.

Un autre but de la présente invention est de fournir un procédé d'utilisation d'un adjuvant de texture du type précédent qui permette d'en maximiser les effets.

Ce but est atteint au moyen d'un procédé dans lequel les ingrédients de l'adjuvant sont ajoutés et/ou mélangés à une température au moins égale à 40°C.

La présence de sel dissocié rend cette contrainte de température très importante.

Le procédé le mieux adapté comporte l'étape de mélange à chaud dans une phase aqueuse des composants de l'adjuvant selon la présente invention.

La température de mélange est avantageusement en présence, fût-elle très faible, de sel dissocié, au moins égale à 50°C, avantageusement à 60°C, de préférence à 80°C.

Il convient aussi de faire en sorte que le mélange ne se fasse pas à température trop élevée. D'une manière générale il est souhaitable que la température n'excède pas la température de transition à laquelle la conformation des macromolécules constituant la gomme xanthane se modifie ; il est préconisé de réaliser l'addition à une température au plus égale à 110°C, avantageusement à 100°C.

Les conditions de cette mise en mélange (temps, pH, etc.) doivent être suffisamment douces pour que l'on évite la dégradation des constituants et notamment des gommes guar et xanthane. Ainsi il est préférable que la durée de mélange ne dépasse pas un quart de journée, avantageusement 2 heures, de préférence 1/2 heure aux températures maximales spécifiées ci-dessus. Les durées peuvent être supérieures si l'on se place dans la partie basse des fourchettes ci-dessus. A titre indicatif la durée minimale de mise en mélange aux températures ci-dessus varie en général entre 1/24 et 1/4 d'heure.

Selon une variante avantageuse de la présente invention, l'addition dudit galactomannane dans la phase aqueuse se fait sous la forme d'une solution aqueuse dudit sel et dudit galactomannane. Selon une autre variante avantageuse de la présente invention, l'addition dudit galactomannane dans la phase aqueuse se fait sous forme solide, ainsi que celle dudit sel sous forme solide. Selon une autre variante avantageuse de la présente invention, l'on introduit dans la phase aqueuse préalablement salée ledit galactomannane sous forme solide.

Pour ce qui est de l'addition dudit xanthane, elle se fait selon diverses variantes avantageuses, qui peuvent se combiner avec toutes les variantes d'addition dudit galactomannane. Ces variantes sont :

. l'introduction dudit xanthane (ou plus exactement ladite gomme xanthane) dans la phase aqueuse sous la forme d'une solution aqueuse dudit sel et dudit xanthane ;
. l'introduction dudit xanthane dans la phase aqueuse et dudit sel sous forme solide ;
. l'introduction dudit xanthane dans la phase aqueuse préalablement salée ladite gomme xanthane sous forme solide.

Il est également possible de réaliser l'addition sous forme de solution des trois ingrédients.

Par phase aqueuse on entend dans la présente description toute phase contenant au moins la moitié d'eau (en masse). Toutefois il est souhaitable que les phases aqueuses contiennent au moins deux tiers avantageusement trois quarts, de préférence cinq sixième d'eau (en masse).

Un autre but de la présente invention est de fournir une composition du type précédent qui présente une texture bien épaisse.

Un autre but de la présente invention est de fournir une composition du type précédent qui soit alimentaire pour des vertébrés supérieurs et notamment pour des mammifères y compris l'homme.

Ces buts et d'autres qui apparaîtront par la suite sont atteints au moyen d'une composition comportant :

. Une phase aqueuse ;
. un galactomannane dont le rapport mannose/galactose est au plus égal à 10/3 ;
. une gomme xanthane ;

dans laquelle ledit galactomannane présente une masse moléculaire telle que sa viscosité intrinsèque $[\eta]$ ([èta]) soit au moins égal à environ 1 m$^3$/Kg.

La présence d'un sel dissocié permet à la présente composition d'être, à une température au moins égale à l'ambiante (environ 20°C), sous la forme d'un gel aqueux. Le gel existe à l'ambiante sans la présence de sel lorsque la viscosité intrinsèque $[\eta]$ ([èta]) dudit galactomannane est au moins égale à 1, 5 m$^3$/Kg.

Au cours de l'étude qui a mené à la présente invention, il a été montré que les compositions selon la présente invention, présentaient de la biréfringence. La valeur de cette biréfringence à concentration de sel égale, (du moins lorsque la mesure est possible) est maximale, ou voisine du maximum, pour les propriétés texturantes les meilleures.

La concentration en galactomannane de telles compositions est avantageusement au moins égale à C*, de préférence C**.

En outre, il est souhaitable que la teneur en masse de la somme du galactomannane et du xanthane soit comprise entre 1 ‰ et 5%, de préférence entre 0,2 et 2 % de la quantité d'eau contenue dans ladite phase aqueuse.

On peut également indiquer qu'il est souhaitable que le rapport entre le sel et les polyosides [c'est-à-dire la somme du galactomannane et du xanthane] soit comprise entre $10^{-5}$·et 0,2 eq(uivalent) de cation (et donc en anion) par gramme de polyoside, avantageusement entre $10^{-4}$ et 0,05 eq de cation (et donc en anion) par gramme de polyoside, de préférence entre $10^{-3}$ et 0,01 eq de cation (et donc en anion) par gramme de polyoside. Cela est valable pour tous les aspects de la présente invention (adjuvant, procédé et composition).

Les résultats sont particulièrement bons lorsque la concentration en sel est telle que la normalité en cation (et donc en anion) soit comprise entre $10^{-3}$ et 0,5, avantageusement entre $5.10^{-3}$ et 0,2.

Parmi les galactomannanes qui répondent aux contraintes spécifiées ci-dessus, on peut citer ceux issus du tara et du guar. Toutefois les effets sont particulièrement marqués dans le cas des galactomannanes issus du guar, que ces derniers soient chimiquement modifiés ou non.

Lorsque l'on veut utiliser des galactomannanes issus de la caroube ou du cassia, pour être conforme à l'invention il faut qu'ils soient triés physiquement, chimiquement modifiés ou plutôt qu'ils soient mélangés avec des galactomannanes issus d'autre provenance, de manière à répondre à la contrainte du rapport M/G.

Parmi les modifications chimiques des galactomannanes issus du guar qui peuvent encore améliorer les résultats, on peut citer notamment les lyses permettant d'augmenter le rapport entre mannose et galactose (mannose/galactose), notamment pour l'amener à des valeurs qui soient au moins égales à 1,5 ; avantageusement au moins égales à 1,8 ; de préférence à 2 ; valeurs qui on déjà été mentionnées plus haut dans la description. Il convient bien entendu de rester dans les limites spécifiées ci-dessus (rapport mannose/galactose au plus égal à 10/3 , plus souvent à 5/2).

Au moyen de l'enseignement de la présente demande, l'homme de métier peut adapter l'invention aux problèmes spécifiques qui lui sont posés, et en particulier choisir les paramètres qui pourront permettre de choisir la viscosité, la température de fusion du gel et/ou la tenue du gel désirée.

La définition de la notion de gel varie souvent selon les auteurs, aussi dans la présente description a-t-on choisi la définition la plus rigoureuse, à savoir la définition scientifique qui considère qu'il y a gel lorsque les courbes des modules G' (module de stockage ou module élastique) et G" (module de perte ou module visqueux) sont indépendantes ou peu dépendantes de la fréquence dans l'intervalle $10^{-2}$ à 1 rad/seconde de la fréquence.

Le pH optimal des compositions selon la présente invention est supérieur à 2 et avantageusement compris entre 3 et 12, de préférence entre 4 et 8.

Les mélanges ci-dessus peuvent notamment servir à la texture :

des cosmétiques ;

des détergents ménagers ;

des boues de forage ;

de spécialités pharmaceutiques (par exemple formulation de sirop en permettant une rhéologie adaptée pour un bon maintien dans la cuillère en conservant l'écoulement du flacon) ;

de compositions alimentaires notamment en substitution de la gélatine.

Les exemples non limitatifs suivants illustrent l'invention.

**Echantillons utilisés**

échantillons de galactomannane et de Xanthane

| Echantillon | Viscosité intrinsèque [η] et constante de Huggins ( $\lambda_1$ ) | Masse moléculaire | rapport M/G |
|---|---|---|---|
| Guar 1 | 1,98 ($\lambda_1 = 0,55$) | $3,3\ 10^6$ | 2,0 |
| Guar 2 | 1,91 ($\lambda_1 = 0,32$) | $3,2\ 10^6$ | 1,7 |
| Guar 3 | 1,59 ($\lambda_1 = 0,33$) | $2,5\ 10^6$ | 1,6 |
| Guar 4 | 1,13 ($\lambda_1 = 0,5$) | $1,5\ 10^6$ | 2,0 |
| Caroube C1 | 1,49 ($\lambda_1 = 0,44$) | $2,2\ 10^6$ | 3,8 |

échantillon de xanthane

| Echantillon | Viscosité intrinsèque [η] et constante de Huggins (λ₁) | acide pyruvique (% sur MS) | taux d'acide pyruvique | acide acétique (% sur MS) | taux d'acétate |
|---|---|---|---|---|---|
| xanthane 1 | 6,5 (λ₁ = 1,09) | 4,5 | 70 % | 5,2 | 100 % |

La température de mélange, sauf lorsque l'on précise, est de 60°C environ.

Les mesures de G' sont réalisées sur un "Rheometrics RFSII®"

**Exemple 1** : effet de la viscosité intrinsèque sur la température de fusion des gels de mélanges galactomannes-xanthane en absence et en présence de sel

Les conditions opératoires sont les suivantes :

. température : variable ;
. fréquence : balayage ;
. concentration totale (guar + xanthane) C = 0,5 % en masse ;
. concentration en sel : variable ;
. échantillon de Guar : G1.

Les résultats sont rassemblés ci après ; les valeurs de G' sont exprimées en Pascals (Pa).

| Mélange testé | température de fusion de gel selon type de gomme guar utilisée définie par son [η] et son rapport mannose/galactose | | | |
|---|---|---|---|---|
| | G4 [η] = 1,13 M/G = 2 | G3 [η] = 1,59 M/G = 1,6 | G2 [η] = 1,91 M/G = 1,7 | G1 [η] = 1,98 M/G = 2 |
| X/G = 10/90 | 37°C | 38°C | 46°C | 46°C |
| X/G = 10/90 KCl = 0,013 M | 45°C | 45°C | 49°C | 50°C |
| X/G = 10/90 KCl = 0,13 M | 58°C | 60°C | 59°C | 60°C |
| X/G = 1 | <<25°C | <<25°C | 28°C | 32°C |
| X/G = 1 KCl = 0,013 M | <<25°C | <<25°C | 38°C | 41°C |
| X/G = 1 KCl = 0,13 M | 43°C | 41°C | 55°C | 56°C |

**Exemple 2** : rôle de la masse moléculaire et du rapport M/G sur le module G' des mélanges galactomannes-xanthane

Les conditions opératoires sont les suivantes :

. température : 25°C ;

. fréquence : 0,1 rad/seconde ;

. concentration totale (guar + xanthane) C = 0,5 % en masse ;

. concentration en sel : 0,13M KCl.

Les résultats relatifs à l'effet de la masse moléculaire sur un domaine plus vaste sont rassemblés dans le tableau ci-après.

| rapport massique en % Xanthane/(guar + xanthane) | Valeur de G' en KCl 0,13 M selon type de gomme guar utilisée définie par son [η] et son rapport mannose/galactose | | |
|---|---|---|---|
| | G1 [η] = 1,98 M/G = 2 | G3 [η] = 1,59 M/G = 1,6 | G2 [η] = 1,91 M/G = 1,7 |
| 0,00 | 0,04 | 0,007 | 0,05 |
| 1,00 | 0,4 | 0,12 | n.d. |
| 2,0 | 1,5 | 0,4 | n.d. |
| 5 | 5,3 | 1,6 | 3 |
| 10 | 10,2 | 2,2 | 6,3 |
| 20 | 25,4 | 3,1 | 9,4 |
| 30 | 28,4 | 2,9 | 13,4 |
| 40 | 30 | 3,2 | 13,3 |
| 50 | 27 | 3,1 | 12,8 |
| 60 | 20,7 | 3,0 | 11,3 |
| 70 | 15,4 | 3,2 | 9,6 |
| 80 | 14,4 | 3,8 | 6,9 |
| 90 | 8,8 | 4,2 | nd |
| 95 | 6,9 | 4,4 | nd |
| 99 | 5,1 | nd | nd |
| 100 | 3,4 | 3,4 | 3,4 |

n.d. = non déterminé

**Exemple 3** : effet du rapport entre xanthane et galactomannanes et rôle du sel sur les mélanges galactomannes-xanthane

Les conditions opératoires sont les suivantes :

. température : 25°C
. fréquence : 0,1 rad/seconde
. concentration totale (guar + xanthane) C = 0,5 % en masse
. concentration en sel : variable
. échantillon de Guar : G1

les résultats sont rassemblés ci après ; les valeurs de G' sont exprimées en Pascals (Pa).

| Rapport massique en % (xanthane/(guar + xanthane)) | Valeur de G' en milieu | | |
|---|---|---|---|
| | non salin | KCl 0,013 M | KCl 0,13 M |
| 0 | 0,03 | 0,02 | 0,04 |
| 10 | 4,4 | 8,6 | 10,2 |
| 20 | 6,0 | 9,9 | 25,4 |
| 30 | 5,7 | 11,2 | 28,4 |
| 40 | 5,6 | 11,6 | 30 |
| 50 | 5,3 | 10,4 | 27 |
| 60 | 4,5 | 7,1 | 20,7 |
| 70 | 3,8 | 4,3 | 15,4 |
| 80 | 2,6 | 4,0 | 14,4 |
| 90 | 2,1 | 2,2 | 8,8 |
| 100 | 1,4 | 1,6 | 3,4 |

On remarque que les effets sont très marqués lorsque le sel passe d'une normalité de 0,01 à 0,1. Les meilleurs effets sont pour des valeurs Xanthane/(guar + xanthane) inférieures à 50 %

**Exemple 4** : Influence de la température de mélange sur le module G'

Les conditions opératoires sont les suivantes :

. température de mélange : variable
. température de mesure : 25 C
. fréquence : 1 rad/seconde
. concentration totale (guar + xanthane) C = 0,5 % en masse
. concentration en sel : variable
. échantillon de Guar : G1

Les résultats sont rassemblés ci après ; les valeurs de G' sont exprimées en Pascals (Pa).

| Température de préparation | G/X = 9 sans sel | G/X = 9 0,013M KCl | G/X = 9 0,13M KCl | G/X = 1 0,013M KCl |
|---|---|---|---|---|
| 25°C | 11,2 | 2,5 | 1,5 | 9,2 |
| 35°C | nd | 2,9 | 1,5 | 9,4 |
| 40°C | 9,7 | 4,2 | nd | nd |
| 45°C | nd | 6,5 | 2,9 | 17,9 |
| 50°C | 9,7 | 9,0 | 5,7 | nd |
| 55°C | nd | 12,3 | 9,3 | 24,6 |
| 60°C | 8,2 | 13,9 | nd | nd |
| 65°C | nd | 10,7 | 10,3$ | 33,3 |
| 70°C | 8,6 | nd | 14,2 | nd |
| 75°C | nd | nd | nd | 27,8 |
| 80°C | 7,3 | 10,2 | 15,2 | 26,7 |
| 90°C | nd | nd | nd | 25,3 |
| 100°C | nd | nd | 11,1 | nd |

**Exemple 5** : température de fusion des mélanges xanthane/ galactomannanes : influence des rapports massiques en % (xanthane/(guar + xanthane)) [X/(G+X)] en présence et en absence de sel

Les conditions opératoires sont les suivantes :

- . température : variable
- . fréquence : balayage
- . concentration totale (guar + xanthane) C = 0,5 % en masse
- . concentration en sel : variable
- . échantillon de Guar : G1.

Les résultats sont rassemblés dans le tableau ci-après.

| Température de fusion en fonction de la force ionique | | | |
|---|---|---|---|
| X/(G+X) | sans sel | 0,013 M KCl | 0,13 M KCl |
| 01 % | 46°C | 51°C | 59°C |
| 05 % | 46°C | 51°C | 60°C |
| 10 % | 46°C | 50°C | 59°C |
| 20 % | 42°C | 49°C | 60°C |
| 30 % | 39°C | 47°C | 58°C |
| 40 % | 36°C | 46°C | 57°C |
| 50 % | 32°C | 41°C | 56°C |
| 60 % | 28°C | 33°C | 53°C |
| 70 % | nd | 25°C | 48°C |
| 80 % | nd | nd | 27°C |
| 90 % | nd | nd | nd |
| 100 % | 43°C | 61°C | 110°C |

On constate que les points de fusion les plus élevés se situent entre 0,5 % et 1/3 de xanthane.

**Exemple 6 (comparatif)** : température de fusion des mélanges xanthane/ galactomannanes cas de la gomme issue de la caroube

| | température de fusion en fonction du milieu testé | | |
|---|---|---|---|
| rapport xanthane/ galactomannanes | eau | 0,013M KCl | 0,13M KCl |
| 10/90 | 56°C (46°C)* | 57°C (50°C) | 59°C (59°C) |
| 50/50 | 53°C (32°C) | 54°C (41°C) | 60°C (56°C) |
| 80/20 | 44°C (-) | 51°C (-) | 58°C (27°C) |

**\* les valeurs entre parenthèses et en italique sont celles correspondant au cas de la gomme guar (G1)**

**Exemple 7** : crème à récurer

Cette crème est composée de particules en dispersion dans une phase aqueuse continue selon l'invention.
La dite invention permet de stabiliser la dispersion en évitant la sédimentation lors du stockage. Néanmoins la rhéologie de type rhéofluidifiant permet une utilisation aisée du produit.
Ces particules qui favorisent le nettoyage des surfaces dures sont à choisir parmi :

- le carbonate de Calcium
- la terre de Diatomée
- la silice.........

Généralement ce type de formulation contient également quelques pour cent [de 1 à 10, mais préférentiellement de 2 à 5 %] de tensio- actifs non ioniques ou amphotères permettant d'améliorer le mouillage et le pouvoir détergent de cette formulation.

**Revendications**

1.   Adjuvant utile comme agent de texture comportant :

   - une gomme xanthane,
   - un galactomannane dont le rapport en équivalent entre les motifs mannose et les motifs galactose (M/G) est au plus égal à 10/3, ledit galactomannane présente une viscosité intrinsèque [η] (éta) d'au moins égale à environ 1,5 m$^3$/kg, et
   - éventuellement un ou un mélange de sel(s) dissocié(s).

2.   Adjuvant selon la revendication 1, caractérisé en ce que le galactomannane présente un rapport mannose/galactose au moins égal à environ 3/2, avantageusement au moins égal à 5/3, de préférence à 1,8, plus prérérentiellement à 2.

3.   Adjuvant selon l'une des revendications 1 ou 2, caractérisé en ce que le galactomannane est le guar.

**4.** Adjuvant selon l'une quelconque des revendications 1 à 3, caractérisé en ce que la gomme xanthane et le galactomannane sont présents dans un rapport massique xanthane/galactomannane (X/G) au moins égal à 1/500, avantageusement à 1/100, de préférence à 1/50.

**5.** Adjuvant selon l'une quelconque des revendications 1 à 4, caractérisé en ce que la gomme xanthane et le galactomannane sont présents dans un rapport massique xanthane/galactomannane (X/G) choisi entre 1/500 et 2, avantageusement entre 1/100 et 1, de préférence à 1/50 et 1/3.

**6.** Adjuvant selon l'une quelconque des revendications 1 à 5, caractérisé en ce que lorsqu'il comporte un ou un mélange de sel(s) dissocié(s), le rapport entre le ou les sel(s) et les polyosides (la somme du galactomannane et du xanthane) est compris entre $10^{-5}$ et 0,2, avantageusement entre $10^{-4}$ et 0,05, de préférence entre $10^{-3}$ et 0,01, équivalent de cation par gramme de polyoside.

**7.** Adjuvant selon l'une quelconque des revendications 1 à 6, caractérisé en ce que dans le sel ou le mélange de sel(s) dissocié(s), l'anion est choisi parmi les halogénures ou leurs mélanges.

**8.** Adjuvant selon l'une quelconque des revendications 1 à 7, caractérisé en ce que dans le sel ou le mélange de sel(s) dissocié(s), le cation est choisi parmi les alcalins ou leurs mélanges.

**9.** Procédé pour texturer une phase aqueuse dans lequel les ingrédients de l'adjuvant sont ajoutés et/ou mélangés dans une phase aqueuse à une température au moins égale à 40°C.

**10.** Procédé pour texturer une phase aqueuse, caractérisé en ce qu'il comporte l'étape de mélange des composants de l'adjuvant selon l'une quelconque des revendications 1 à 8, y compris le(s) sel(s) dissociée(s), dans une phase aqueuse à une température au moins égale à 50°C.

**11.** Procédé selon l'une des revendications 9 ou 10, caractérisé par le fait que la température de l'étape de mélange est au plus égale à 110°C, avantageusement égale à 100°C.

**12.** Composition comportant :

- une phase aqueuse,
- une gomme xanthane,
- un galactomannane dont le rapport mannose/galactose est au plus égal à 10/3, présentant une viscosité intrinsèque [η] (éta) égale à environ 1,5 m$^3$/kg,

ladite composition se présentant sous forme d'un gel aqueux à une température au moins égale à environ 20°C.

**13.** Composition selon la revendication 12, caractérisée par le fait qu'elle se présente sous forme d'un gel aqueux à une température au moins égale à environ 20°C, lorsqu'elle comporte un sel dissocié.

**14.** Composition selon la revendiation 13, caractérisée par le fait que la concentration en sel est telle que la normalité en cation soit comprise entre $10^{-3}$ et 0,5, avantageusement entre $5.10^{-3}$ et 0,2.

**15.** Utilisation de l'adjuvant selon l'une quelconque des revendications 1 à 8, comme agent de texture et/ou de gélification de phase aqueuse.


**Patentansprüche**

**1.** Zusatzstoff, der als Strukturbildungsmittel geeignet ist, umfassend:

- einen Xanthangummi,
- ein Galactomannan, dessen Äquivalentverhältnis zwischen Mannosemotiven und Galactosemotiven (M/G) höchstens gleich 10/3 ist, wobei das Galactomannan eine Grundviskosität [η] (eta) von mindestens ungefähr 1,5 m$^3$/kg aufweist, und
- gegebenenfalls ein dissoziiertes Salz oder eine Mischung von dissoziierten Salzen.

**2.** Zusatzstoff nach Anspruch 1, dadurch gekennzeichnet, daß das Galactomannan ein Mannose/Galactose-Verhältnis von mindestens ungefähr 3/2, vorteilhafterweise mindestens 5/3, vorzugsweise mindestens 1,8, noch bevorzugter mindestens 2 aufweist.

**3.** Zusatzstoff nach einem der Ansprüche 1 oder 2, dadurch gekennzeichnet, daß das Galactomannan Guarmehl ist.

**4.** Zusatzstoff nach einem der Ansprüche 1 bis 3, dadurch gekennzeichnet, daß der Xanthangummi und das Galactomannan in einem Massenverhältnis Xanthan/Galactomannan (X/G) von mindestens 1/500, vorteilhafterweise von mindestens 1/100, vorzugsweise von mindestens 1/50 vorliegen.

**5.** Zusatzstoff nach einem der Ansprüche 1 bis 4, dadurch gekennzeichnet, daß der Xanthangummi und das Galactomannan in einem Massenverhältnis Xanthan/Galactomannan (X/G) ausgewählt zwischen 1/500 und 2, vorteilhafterweise zwischen 1/100 und 1, vorzugsweise von 1/50 und 1/3 vorliegen.

**6.** Zusatzstoff nach einem der Ansprüche 1 bis 5, dadurch gekennzeichnet, daß, wenn er ein dissoziiertes Salz oder eine Mischung von dissoziierten Salzen umfaßt, das Verhältnis zwischen dem oder den Salz(en) und den Polyosiden (der Summe des Galactomannans und des Xanthans), bezogen auf Kation-Äquivalent pro Gramm Polyosid, zwischen $10^{-5}$ und 0,2, vorteilhafterweise zwischen $10^{-4}$ und 0,05, vorzugsweise zwischen $10^{-3}$ und 0,01 beträgt.

**7.** Zusatzstoff nach einem der Ansprüche 1 bis 6, dadurch gekennzeichnet, daß in dem dissoziierten Salz oder der Mischung von dissoziierten Salz(en) das Anion aus den Halogeniden oder deren Mischungen ausgewählt ist.

**8.** Zusatzstoff nach einem der Ansprüche 1 bis 7, dadurch gekennzeichnet, daß in dem dissoziierten Salz oder der Mischung von dissoziierten Salz(en) das Kation aus den Alkalimetallen oder deren Mischungen ausgewählt ist.

**9.** Verfahren zur Strukturbildung in einer wäßrigen Phase, in welchem die Bestandteile des Zusatzstoffs bei einer Temperatur von mindestens 40°C einer wäßrigen Phase zugesetzt oder in eine wäßrige Phase eingemischt werden.

**10.** Verfahren zur Strukturbildung in einer wäßrigen Phase, dadurch gekennzeichnet, daß es den Schritt umfaßt, die Komponenten des Zusatzstoffs nach einem der Ansprüche 1 bis 8, welcher das oder die dissoziierte(n) Salz(e) umfaßt, in eine wäßrige Phase bei einer Temperatur von mindestens 50°C einzumischen.

**11.** Verfahren nach einem der Ansprüche 9 oder 10, dadurch gekennzeichnet, daß die Temperatur des Einmischschritts höchstens 110°C, vorteilhafterweise höchstens 100°C beträgt.

**12.** Zusammensetzung, umfassend:

- eine wäßrige Phase,
- einen Xanthangummi,
- ein Galactomannan, dessen Mannose/Galactose-Verhältnis höchstens 10/3 ist und das eine Grundviskosität [η] (eta) von ungefähr 1,5 m³/kg aufweist,

wobei die Zusammensetzung bei einer Temperatur von mindestens ungefähr 20°C in Form eines wäßrigen Gels vorliegt.

**13.** Zusammensetzung nach Anspruch 12, dadurch gekennzeichnet, daß sie bei einer Temperatur von mindestens ungefähr 20°C in Form eines wäßrigen Gels vorliegt, wenn sie ein dissoziiertes Salz enthält.

**14.** Zusammensetzung nach Anspruch 13, dadurch gekennzeichnet, daß die Konzentration an Salz so ist, daß die Normalität an Kationen zwischen $10^{-3}$ und 0,5, vorteilhafterweise zwischen $5 \cdot 10^{-3}$ und 0,2 ist.

**15.** Verwendung des Zusatzstoffs nach einem der Ansprüche 1 bis 8 als Strukturbildungsmittel und/oder Geliermittel für wäßrige Phasen.

**Claims**

1. Adjuvant of use as texturizing agent comprising:

   - a xanthan gum,
   - a galactomannan, the ratio of which in equivalents between the mannose units and the galactose units (M/G) is at most equal to 10/3, the said galactomannan exhibiting an intrinsic viscosity [η] (eta) of at least equal to approximately 1.5 m$^3$/kg, and
   - optionally a or a mixture of dissociated salt(s).

2. Adjuvant according to claim 1, characterized in that the galactomannan exhibits a mannose/galactose ratio at least equal to approximately 3/2, advantageously at least equal to 5/3, preferably to 1.8, more preferably to 2.

3. Adjuvant according to either of claims 1 and 2, characterized in that the galactomannan is guar.

4. Adjuvant according to any one of claims 1 to 3, characterized in that the xanthan gum and the galactomannan are present in a xanthan/galactomannan (X/G) ratio by mass at least equal to 1/500, advantageously to 1/100, preferably to 1/50.

5. Adjuvant according to any one of claims 1 to 4, characterized in that the xanthan gum and the galactomannan are present in a xanthan/galactomannan (X/G) ratio by mass chosen between 1/500 and 2, advantageously between 1/100 and 1, preferably between 1/50 and 1/3.

6. Adjuvant according to any one of claims 1 to 5, characterized in that, when it comprises a or a mixture of dissociated salt(s), the ratio of the salt(s) to the polyglycosides (the sum of the galactomannan and xanthan) is between 10$^{-5}$ and 0.2, advantageously between 10$^{-4}$ and 0.05, preferably between 10$^{-3}$ and 0.01, cation equivalent per gram of polyglycoside.

7. Adjuvant according to any one of claims 1 to 6, characterized in that, in the dissociated salt or mixture of dissociated salts, the anion is chosen from halides or their mixtures.

8. Adjuvant according to any one of claims 1 to 7, characterized in that, in the dissociated salt or mixture of dissociated salts, the cation is chosen from alkali metals or their mixtures.

9. Process for texturizing an aqueous phase, in which process the ingredients of the adjuvant are added to and/or mixed in an aqueous phase at a temperature at least equal to 40°C.

10. Process for texturizing an aqueous phase, characterized in that it comprises the stage of mixing the components of the adjuvant according to any one of claims 1 to 8, including the dissociated salt(s), in an aqueous phase at a temperature at least equal to 50°C.

11. Process according to either of claims 9 and 10, characterized in that the temperature of the mixing stage is at most equal to 110°C, advantageously equal to 100°C.

12. Composition comprising:

    - an aqueous phase,
    - a xanthan gum,
    - a galactomannan, the mannose/galactose ratio of which is at most equal to 10/3, exhibiting an intrinsic viscosity [η] (eta) equal to approximately 1.5 m$^3$/kg,

    the said composition existing in the form of an aqueous gel at a temperature at least equal to approximately 20°C.

13. Composition according to claim 12, characterized in that it exists in the form of an aqueous gel at a temperature at least equal to approximately 20°C when it comprises a dissociated salt.

14. Composition according to claim 13, characterized in that the concentration of salt is such that the cation normality is between 10$^{-3}$ and 0.5, advantageously between $5 \times 10^{-3}$ and 0.2.

**EP 0 861 291 B1**

**15.** Use of the adjuvant according to any one of claims 1 to 8 as texturizing and/or gelling agent for an aqueous phase.